# EUROPEAN PATENT APPLICATION

(11) **EP 1 900 379 A2**
(43) Date of publication of application: **19.03.2008**
(21) Application number: 07123000.7
(22) Date of filing: 10.11.2003
(51) Int. Cl.: A61K 47/48

(54) **Method for the selective and quantitative functionalization of immunoglobulin FAB fragments, conjugate compounds obtained with the same and composition thereof**

(30) Priority: 13.11.2002 EP 02025315
(62) Divisional of application: 03779875.8
(71) Applicant: Bracco Imaging, S.P.A., 20134 Milano (IT)
(72) Inventor: De Haen, Christoph, 20134, Milano (IT); Maisano, Federico, 20134, Milano (IT)
(74) Representative: Poletti, Marco

(57) **Abstract**

The invention provides chemical conjugates between an immunoglobulin Fab fragment and molecular entities imparting diagnostic or therapeutic utility, whereby the only sites of conjugation on the Fab fragment are one or both of the sulfhydryl groups deriving from the selective and quantitative reduction of the inter-chain disulfide bond of said Fab fragment and whereby said molecular entities imparting diagnostic or therapeutic utility have at least one free sulfhydryl-reactive group, characterized in that the conjugation stoichiometric molar ratio molecular entity to Fab fragment is in the range from 0.95 to 1.05 or in the range from 1.95 to 2.05.

The invention also provides a process for preparing said conjugates and pharmaceutical compositions thereof.

## Description

### FIELD OF THE INVENTION

This invention relates to conjugates of immunoglobulin Fab fragments (Fab), in which said Fab have been quantitatively and selectively funzionalized only at predetermined specific desired sites of the molecule.

The invention also relates to a method for obtaining said selective and quantitative functionalization, as well as to pharmaceutical compositions comprising said conjugates.

### BACKGROUND OF THE INVENTION

Monoclonal antibodies (mAb) are proteins with the well-known capability to localise both *in vitro* and *in vivo* on cells or on tissues which expose the antigen to which they are specific. This property is maintained in some of their well-known proteolytic fragments, e.g. Fab, Fab' and F(ab')₂. In particular, immunoglobulin Fab fragments (hereafter also in the plural simply called Fab) maintain this property.

It is well known that diagnostic or therapeutic molecules of different type, or precursors thereof, may be covalently linked to a mAb or its fragments. Those conjugates in which the linked diagnostic or therapeutic molecule does not interfere with the capability of binding to the target antigen are able to transport and thus target the molecule to antigen-bearing cells and tissues, where it can exert its intended purpose, such as, for example, diagnostic signals production or therapeutic cell killing.

Fab fragments are of particular interest as diagnostic or therapeutic agents, since they are smaller than intact immunoglobulins or some of their other fragments, e.g. F(ab')₂. Smallness increases their rate of passage from the blood to the tissue interstitium, where many of them find their target. It also increases their diffusivity in the tissue interstitium, and thereby it facilitates and accelerates their arrival at the target site and the disappearance of unbound molecules from said site. Moreover it increases their rate of excretion, thus favouring the reduction of non-specific background effects.

Only minimally larger than Fab fragments are Fab' fragments. These are obtained from F(ab')₂ fragments by reduction of the disulfide bridge linking the two heavy chains, and need to be stabilized by chemical modification of the free sulfhydryl groups.

Many methods for conjugating suitable diagnostic or therapeutic molecules to mAb and their various fragments have already been described. Typically the conjugating molecule modifies the mAb or its fragments at various sites, including some that interfere with binding to antigen. Major loss of binding to antigen can often be achieved by a low stoichiometric ratio of conjugating molecule to protein. Especially for radiodiagnostic purposes stoichiometric ratios substantially below one are sometimes, although not always, acceptable. When they are acceptable, the explanation is found on the one hand in the elevated number of antigenic sites relative to the number of mAb or its fragments necessary for satisfactory signal generation, and on the other hand in the fact that elevated concentrations of the mAb or its fragment have no detrimental pharmacological activity. In these particular cases excesses of unlabeled mAb or its fragments do not significantly interfere with binding of their radiolabeled conjugates and, from a signal-generating standpoint, they are silent.

In contrast, when the number of antigenic sites is very low, or when the mAb or its fragments have detrimental pharmacological activities, it is highly preferable that most or all mAb or fragments are radiolabeled.

The requirement for an elevated fraction, preferably the totality, of mAb or fragments being radiolabeled is particularly pronounced in the case of radiotherapy. In this case the number of antigenic binding sites is almost always limiting therapeutic efficacy. The same requirement holds when the mAb or its fragment has detrimental pharmacological activity, which needs to be contained. In these cases occupation of any sites by unlabeled mAb or fragment is to be avoided.

Elevated stoichiometric ratios of conjugating molecules to protein can easily be achieved for example by chemical modification of free amino groups (amino-terminal α-amino groups and the ε-amino groups of lysines) or of free carboxyl groups (carboxy-terminal α-carboxy groups and the γ-, respectively δ-carboxy groups, of aspartic and glutamic acid). Unfortunately, most of the times this is accompanied by the creation of a substantial fraction of conjugates that binds no more or poorly to antigen. When radiolabeled, such conjugates add noise instead of signal to diagnostic procedures and add radiation load without concomitant therapeutic benefits to therapeutic regimens. Moreover, the described chemical conjugation methods are non-specific for selected sites of the protein and consequently are not useful for obtaining final products in which the numbers and/or the types of the sites of conjugation on the protein or protein fragment are known and well defined. Rather, conjugation randomly occurs on a plurality of reactive and poorly defined sites. As a result, also the stoichiometry of the conjugation products, i.e. the molar ratio diagnostic/therapeutic moiety to protein/protein fragment, results poorly defined. At best only a mean stoichiometric molar ratio of conjugating molecule to protein can be measured and fractional occupancy of certain amino acid residues can be estimated. The actual final product generally consists of a complex, poorly defined mixture of variously substituted compounds, each one having its stoichiometry of substitution. Clinical application of such mixtures of conjugated products is at variance with the norms regarding classical pharmaceutical products. Therefore health regulatory bodies are calling for chemically better defined conjugates involving immunoglobulins and/or their fragments.

Such products, even if highly needed, have so far not been practical, since by present means they are obtained in low yields and require costly and industrially impractical separation methods. It would thus be highly desirable to find a method that allows conjugation of diagnostic or therapeutic molecules to mAb or its fragments at a stoichiometric ratio of at least one, and only at well-defined sites that do not interfere with binding to antigens. The present invention offers a solution to this problem, preferably with regard to Fab fragments.

Fab fragments contain four intra-polypeptide-chain disulfide bridges and one inter-polypeptide-chain disulfide bridge. The single inter-chain disulfide bridge is located close to the carboxy-terminal of the two polypeptide chains, i.e. at the opposite end of the site on the molecule which is responsible for antigen binding. Chemical modifications at this site are therefore expected to have minimal effects on affinity for antigens. Accordingly, realizing a method able to selectively functionalize only said inter-chain disulfide bond leaving untouched the other four intra-chain disulfide bonds, as well as the other possible reactive groups in the molecule, is of the greatest importance for obtaining highly pure and structurally well definied compounds.

Disulfide bridges in proteins can be reduced to pairs of free sulfhydryl groups. Most often that is accomplished by exposing the protein to a very large molecular excess of small-molecular-weight sulfhydryl compounds, such as, for example, mercaptoethanol, dithiothreitol, dithioerythritol, cysteine or glutathione. Under these conditions, disulfide bonds are formed among the small-molecular-weight sulfhydryl compounds, while the protein disulfide bridges are reduced to free sulfhydryl groups. It is difficult to eliminate the excess of reducing sulfhydryl compounds and their oxidation products at the end of the reaction without causing substantial reoxidation of the protein sulfhydryl groups to disulfide bridges again. Thus, usually, when a subsequent sulfhydryl-specific chemical modification of the protein sulfhydryl groups is desired, the modifying agent is added in the continued presence of the excess of the small-molecular-weight sulfhydryl compounds and of their oxidation products. Since all the sulfhydryl groups in the reaction mixture, both those on the protein and those on the small-molecular-weight sulfhydryl compounds, undergo the same reaction with the modifying agent, the latter must be added in large excess over the number of the protein sulfhydryl groups, actually somewhat in excess over the sulfhydryl groups of the small-molecular-weight sulfhydryl compounds. When expensive modifying agents are at stake, this type of process results non-practical from the industrial point of view. Moreover, such conditions are difficult to fully standardise and, worse of all, do not consent to obtain specific and stoichiometrically well defined modifications at the desired sulfhydryl group/groups of the protein or protein fragment of interest, leaving untouched the other ones. In other words, this type of reaction involves, at least partially, all the disulfide groups of the protein, and sometimes also other reactive groups of the same, thus giving a mixture of randomly reacted and unreacted sulfhydryl groups.

The present invention mainly focuses on conjugates involving Fab fragments, substantially because of their size.

As already mentioned, a lot of literature, also comprising patents and patent applications, exists, dealing with the preparation of conjugates of immunoglobulins and their fragments with suitable diagnostic or therapeutic mojeties. Actually none of said documents solves, nor gives useful suggestions for solving, the problem represented by the need of administering to the patient pharmaceutical formulations containing, as active ingredients, Fab fragments which have been selectively and quantitatively funzionalized only at desired specific sites of the molecule, thus showing a predefined, precise substitution stoichiometry.

EP-A-131836, for instance, discloses S-alkylated Fab or Fc fragments of human immunoglobulins (IgG) obtained by reducing the multiple inter-chain disulfide bonds with excess of mercaptoethanol, dithiothreitol or dithioerythritol followed by alkylation of the resulting sulfhydryl groups. However, the disclosed method does not allow a precise control of the stoichiometry of the conjugation on the antibody fragments, thus giving a complex mixture of the various possible products. Moreover, the excess of reducing agent makes the use of a large excess of alkylating agent necessary.

A similar method is disclosed in US 5,612,016 and is applied only to intact IgG or to F(ab')₂ fragments. Reduction of the disulfides is performed with excess of thiol derivatives and can involve more than one disulfide group. The final conjugate may contain at least one ligand per antibody or F(ab')₂ fragment, but different ratios are equally allowed. No mention to a precise, well defined conjugation stoichiometry, as well as to a precise site of conjugation is reported. Also in this case large excess of alkylating agent is required to overcome the excess of reducing agent.

US 5,274,119 shows that selective reduction of inter-chain disulfide bridges of F(ab')₂ fragments with dithiothreitol is possible only under very strictly controlled conditions, e.g. at pH = 7. Moreover subsequent purification over a GF-250 HPLC column is mandatory. That means that the method is not applicable on industrial scale and that at least some of the free sulfhydryl groups are reconverted to disulfide bonds.

Other conjugates of antibodies or fragments thereof, obtained by reaction of the free sulfhydryl groups, deriving from the reduction of disulfide bridges, with different modifiers of the sulfhydryl groups, are disclosed in EP-A-453082, US 4,741,900, EP-A-417927, EP-A-023779, EP-A-332022, EP-A-453082, EP-A-277088, US 5,082,930. However, all these documents generally disclose chemistries which produce mixtures of products of non-defined structure; none of them allows or discloses or, directly or indirectly, teaches the preparation of specifically substituted conjugates characterized by a pre-determined and substantially controlled conjugation stoichiometry.

Very recently, in Bioconjugate Chem, 2001, 12, 178-185, Fab fragments were described in which the reduction of the disulfide bonds was performed by using 2-mercaptoethanol. An illustration of a Fab modified on a C-terminal sulfhydryl group was shown. However, the described reduction introduced 3.67 thiol groups per molecule of Fab, thus leading, even in this case, to conjugates of ill-defined conjugation stoichiometry. It further confirms that the use of thiol derivatives as reducing agents does not represent the solution to the need of obtaining the desired selective reduction of Fab fragments and the corresponding stoichiometrically well defined conjugation.

In the case of Fab fragments, which bear the antigen-binding sites, the problem of selective conjugation at only the C-terminal sulfhydryl groups is intensely perceived by researchers, but cannot be addressed by the methods used for intact immunoglobulins. Despite of this evident need, no other methods have been so far described that are able to specifically and quantitatively direct the conjugation reaction only to the sulfhydryl groups which do not take part in stabilizing the folding of the polypeptide chain, i.e. the two deriving from the inter-chain disulfide bond.

The use of particular phosphine derivatives, such as tributylphosphine or tris-(carboxyethyl)phosphine (TCEP), as reducing agents for the disulfide bonds in proteins has already been disclosed in a number of papers: for example in Methods in Enzymol. 1977, 47, 116-122, J. Org. Chem. 1991, 56, 2648-2650; Eur. J. Nucl. Med. 1995, 22, 690-698, Biophisical Journal 1998, 74, A179, abstr.Tu-Pos196, Faseb Journal 1997, 11, A1361, abstr. 2948, Eur. J. Nucl. Med. 1999, 26, 1265-1273, Anal. Biochem. 1999, 273, 73-80; Protein Science, 1993, 2, 1749-1755; nevertheless, said phosphine agents have never been suggested as possible selective reducing agents for the inter-chain disulfide bonds in Fab and Fab' fragments.

Despite the general teaching of the art, we have now surprisingly found that it is possible to prepare, easily and with convenient yields, conjugates of Fab fragments with diagnostic or therapeutic agents, or useful precursors thereof, which are characterized, within narrow limits of error, by exact conjugation stoichiometries, i.e. showing a conjugation molar ratio agent/agents to Fab of 1:1 or 2:1, being the Fab selectively functionalized only at one or two specific sulfhydryl groups in a predefined position of the Fab, i.e. those deriving from the selective reduction of the disulfide inter-chain bond.

### SUMMARY OF THE INVENTION

In a first preferred embodiment, the present invention provides a chemical conjugate between an immunoglobulin Fab fragment and molecular entities imparting diagnostic or therapeutic utility, whereby the only sites of conjugation on the Fab fragment are one or both of the sulfhydryl groups deriving from the selective and quantitative reduction of the inter-chain disulfide bond of said Fab fragment and whereby said molecular entities imparting diagnostic or therapeutic utility have at least one free sulfhydryl-reactive group, characterized in that the conjugation stoichiometric molar ratio molecular entity to Fab fragment is in the range from 0.95 to 1.05 or in the range from 1.95 to 2.05.

### DETAILED DESCRIPTION OF THE INVENTION

The conjugate is obtained by selectively and quantitatively reducing only the inter-chain disulfide bond of a Fab fragment and then quantitatively functionalizing one of the two obtained sulfhydryl groups by reaction with a first molecular entity which has at least one free sulfhydryl-reactive group and gives therapeutic or diagnostic utility, then, if desired, quantitatively functionalizing also the other sulfhydryl group of the Fab with a second molecular entity having at least one free sulfhydryl-reactive group and imparting diagnostic or therapeutic utility, said second moiety being identical to the first one or even different, in this case possibly giving also different diagnostic or therapeutic properties.

Alternatively and preferably, after reduction of the inter-chain disulfide bond, it is possible to quantitatively obtain the symmetrically diconjugated product by directly reacting said reduced Fab fragment with a stoichiometric excess of one of said conjugating moieties.

The term " quantitatively functionalizing ", as used in this disclosure, means that the final conjugated compounds show:
a) a molar ratio between conjugating molecular entity and Fab fragment ranging from 0.95 to 1.05, when only one of the two free sulfhydryl groups of the reduced Fab is conjugated,
b) a molar ratio between conjugating molecular entity and Fab fragment ranging from 1.95 to 2.05, when both of the two sulfhydryl groups are conjugated either asymmetrically or symmetrically.

In case only one of the sulfhydryl groups deriving from the selective and quantitative reduction of the inter-chain disulfide bond of the Fab is desired as a conjugated, the other one sulfhydryl group may be kept as a free sulfhydryl group or, in turn, may be functionalized with a bocking group. This blocking group preferably comprises a chemical moiety non-imparting diagnostic or therapeutic utility, being said chemical moiety preferably selected among protective groups of the thiol group or small alkylating or arylating agents.

Without thereby limiting the generality of the invention, preferred examples of first molecular entities having a sulfhydryl-reactive group and imparting diagnostic or therapeutic utility comprise suitable derivatives of chelating agents for, or chelates of, radionuclides, paramagnetic metal ions or luminescent metal ions, a chromophoric fluorescent or a phosphorescent molecule, a biotin molecule, a hapten recognized by a distinct antibody or fragment thereof, an avidin or streptavidin molecule, a therapeutic drug, a lipophilic chain bearing molecular entity incorporated into liposomes, phospholipid-stabilized microbubbles, triglyceride- or polymer-based microspheres, microballoons which carry the diagnostic or therapeutic agent. Said first moiety may further comprise one or more functional groups which may be used, as such or after deprotection or after chemical modification, as targets for the selective attachment of a second Fab fragment, equal or different from the first one, or of a second molecular entity imparting diagnostic or therapeutic utility.

Without thereby limiting the generality of the invention, preferred examples of suitable sulfhydryl-reactive groups comprise iodoacetyl, bromoacetyl, vinyl or maleimido groups, or polyfluorobenzene or dinitrofluorobenzene derivatives. If desired, a reversible linkage can be obtained by reaction with another disulfide-containing molecule and formation of mixed disulfides.

The second molecular entity can be the same as the first one or it may be different, thus giving a combination of different residues and, possibly, of different diagnostic or therapeutic effects or even of a mixed diagnostic and therapeutic use.

Preferred examples of said second molecular entity having a sulfhydryl-reactive group and imparting diagnostic or therapeutic utility comprise suitable derivatives of chelating agents for, or chelates of, radionuclides, paramagnetic metal ions or luminescent metal ions, a chromophoric fluorescent or a phosphorescent molecule, a biotin molecule, a hapten recognized by a distinct antibody or fragment thereof, an avidin or streptavidin molecule, a therapeutic drug, a lipophilic chain bearing molecular entity incorporated into liposomes, phospholipid-stabilized microbubbles, triglyceride- or polymer-based microspheres, microballoons which carry the diagnostic or therapeutic agent. Said second moiety may further comprise one or more functional groups which may be used, as such or after deprotection or after chemical modification, as targets for the selective attachment of a second Fab fragment, equal or different from the first one, or of a second molecular entity imparting diagnostic or therapeutic utility.

Even in this case preferred examples of suitable sulfhydryl-reactive groups comprise iodoacetyl, bromoacetyl, vinyl or maleimido groups, or polyfluorobenzene or dinitrofluorobenzene derivatives. If desired, a reversible linkage can be obtained by reaction with another disulfide-containing molecule and formation of mixed disulfides.

Fab fragments are obtained by known methods: the use of rFab, i.e. Fab obtained through recombinant DNA techniques, is particularly preferred.

According to another preferred embodiment, the invention provides a process for the preparation of said conjugates, said process comprising:
a) the selective and quantitative reduction of the inter-chain disulfide bond of a Fab fragment to give two free sulfhydryl groups;
b) the quantitative functionalization of one or both of the sulfhydryl groups from step a) with molecular entities having at least one free sulfhydryl-reactive group and imparting diagnostic or therapeutic utility, to give mono- or diconjugate compounds, said diconjugates deriving from either symmetric or asymmetric functionalization of the sulfhydryl groups.

As above disclosed, a number of reducing agents are known which can be employed for the reduction of disulfide bonds, but, in the present case, specific reagents and specific reaction conditions were needed in order to quantitatively reduce only the inter-chain disulfide bond of a Fab fragment, leaving the other disulfide bonds unaffected. Namely, it is well known in the art that reducing agents for the disulfide bond may be selected from borohydrides, cyanoborohydrides, phosphines, thiol compounds, stannous ions, ascorbate and dithionite. However, none of them has been till now disclosed as a specific reducing agent for the inter-chain disulfide bond od a Fab fragment.

Unexpectedly, phosphines resulted highly promising for reaching this scope, in particular tributylphosphine and tris-(carboxyethyl)phosphine. The last one, hereinafter shortly named with the acronym TCEP, resulted the reducing agent of choice, surprisingly allowing to obtain the desired quantitative and selective reduction only of the inter-chain disulfide bond in Fab fragments, while leaving unaffected the other four -S-S- intra-chain bonds. This goal was obtained by using controlled working conditions and a substantially lower excess of the reducing agent in comparison to other possible reducing compounds. Moreover, no interactions usually happened with the conjugating moieties, so it was also possible to limit the excess of the same during the following condensation step. As a result, less reactants were used, less by-products were formed, no need for intermediate purification of the reduced Fab fragments existed, higher yields of purer, easier to purify, final compounds were obtained.

The preferred found experimental conditions under which the selective and quantitative reduction of the invention takes place are shortly summarized in the following and further detailed in the experimental section.

After mixing the reacting species under buffered conditions (every type of buffer giving the desired pH range is equally usable) according to the teaching of Examples 1 and 3, a final buffered aqueous reaction solution having the following characteristics is obtained:
Fab concentration: 1-100 µM, preferably 1.5-10 µM, most preferably 2-5 µM;
Phosphine concentration: 0.1-10 mM, preferably 0.5-5 mM;
pH of the buffered solution: between 4 and 8, preferably between 5 and 7.
Reaction time ranges from 5 to 180 min, preferably from 25 to 70 min.
Reaction temperature is kept from 4 to 45°C, preferably from 25 to 40°C.

The condensation reaction is usually performed immediately at the end of the reduction of the disulfide bond, in the same reaction medium, by adding a buffered aqueous solution of the desired conjugating molecular entity, without previously purifying the reduced Fab fragment.

The preferred found condensation conditions are disclosed in detail in the experimental section, Examples 1, 4 and 6. The final buffered aqueous reaction solution (every type of buffer giving the desired pH range is equally usable) preferably has the following characteristics:
Fab concentration: 2-5 µM;
Phosphine concentration: 0.5 - 5 mM;
conjugating moiety concentration: 0.1-100 mM;
pH of the buffered solution: between 5 and 7.
Reaction time is preferably ≥ 30 min.
Reaction temperature is kept from 4 to 45°C, preferably from 20 to 40°C.

For the purpose of confirming the nature of the conjugates of the present invention, in particular the stoichiometry of the condensation reaction, we have conjugated a recombinant anti-*Herpes simplex* virus Fab fragment (prepared according to: Cattani P, Rossolini GM, Cresti S, Santangelo R, Burton DR, Williamson RA, Sanna PP, Fadda G; J Clin Microbiol. 1997 Jun; 35(6): 1504-9. "Detection and Typing of Herpes Simplex Viruses by Using Recombinant Immunoglobulin Fragments Produced in Bacteria"), selectively reduced at the inter-chain disulfide bond by using the method of the invention, with β-maleimidopropionic acid, as disclosed in Example 1. This last molecule adds a carboxylate residue to the free sulfhydryl group and thus enables the measurement of the number and type of conjugated molecules by using a simple ion-exchange chromatography method.

The alkylation reaction was performed immediately at the end of the reduction of the disulfide bond, in the same reaction medium, without purifying the reduced Fab fragment.

Said alkylation reaction was performed under the preferred found conditions of the invention as fully disclosed in Example 1.

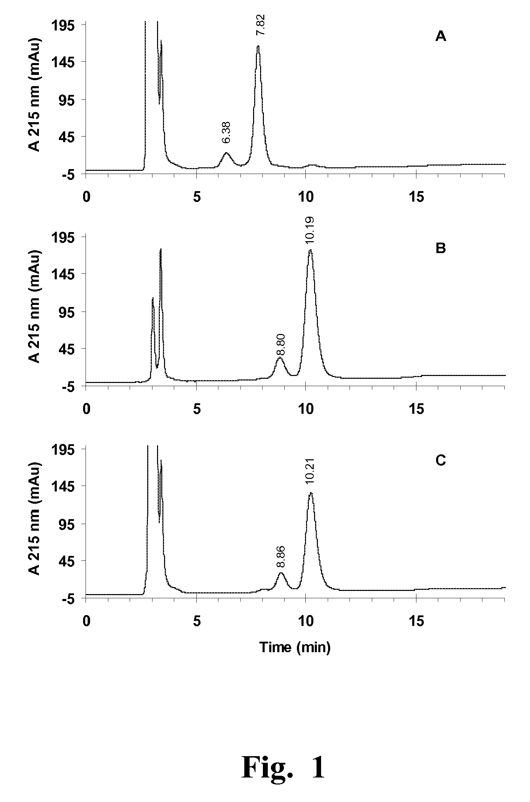

At the end of the reaction, it was possible to calculate the total number of added carboxylate groups, in this case confirming that both of the two free sulfhydryl group underwent the conjugation reaction, as also shown in Fig.1.

The reaction conditions may vary according to the reactivity of the various thiol-reactive molecular entities, to their molecular weight and steric hindrance, to the desired final compound (mono- or di-conjugated, symmetrically or not) and it is generally advisable to control that, if the reduction step is omitted, no lateral reaction occurs (this confirms that only the two sulfhydryl groups deriving from the reduction of the single inter-chain disulfide bridge of the Fab react with the conjugating moiety/ies).

It is particularly preferable to perform the alkylation reaction without previous separation of the excess of reducing agent, because the inter-chain disulfide bond can easily reform.

The conjugate compounds of the invention are particularly advantageous because:
a pre-determined, controlled stoichiometry of conjugation greatly reduces the percentage of residual impurities, which can be inactive or inhibitory, or even toxic, in the final compound;
the products are easily characterised and characterizable for drug
registration purposes before the health authorities;
the process for the preparation of the products is relatively easy, has good yields and is applicable on industrial scale;
the purification of the final diagnostic or therapeutic compounds, or of
their precursors, results simple because it implies the separation of mixtures mainly containing products with 0, 1 or 2 substituents, said mixtures being greatly enriched in only one of them;
the conjugated moieties are exclusively located near the carboxy
terminal of the Fab heavy and light chains, therefore they are not likely to interfere with the antigen recognition site, which is formed by
residues near the amino terminal part of the polypeptide chains;
the initial conformation of the Fab is maintained.

Fab conjugates according to the invention will usually be directed against antigens of therapeutic or diagnostic interest, e.g. against tumor antigens, receptors, tissue markers, markers for specific pathologies, infections, inflammations, degenerative processes and so on.

So, according to a further preferred embodiment, the invention also provides diagnostic and/or therapeutic compositions containing said conjugates as active ingredients.

For the desired diagnostic or therapeutic applications, the conjugate compounds of the invention will be formulated in suitable compositions, usually in the form of suspensions, solutions or emulsions for parenteral administration, lyophilizates to be reconstituted before use or even in the form of other pharmaceutical compositions suitable for other desired different types of administration. The dose will depend on several parameters (kind of ligand, patient's conditions) but it will generally be in the range from 0.1 to 10 mg of conjugate per single administration in the case of diagnostic applications and in the range from 10 to 500 mg of conjugate per single administration in the case of therapeutic applications.

The conjugate compounds of the invention are particularly advantageous also for their *in vitro* use, whereby they show their utility, preferably when applied to immunochemical tests *in vitro.*

The invention equally applies to Fab' fragments, which, as previously mentioned, have structure similar to and dimension not much larger than Fab.

### EXPLAINATION OF THE FIGURES

Fig. 1 shows cation-exchange HPLC analyses of reaction mixtures between the rFab of Example 1 and β-maleimidopropionic acid. In all runs, the peaks eluting before 5 min are due to salts and reactants that absorb at 215 nm.
   A) is the complete reaction mixture, containing both TCEP and β-maleimidopropionic acid;
   B) is the initial unreacted rFab solution;
   C) is the incomplete reaction mixture, without TCEP, but containing β-maleimidopropionic acid.

   A) shows that the initial rFab has completely reacted, giving a unique conjugation compound. Mass Spectrometry (MS) analysis demonstrated that the disubstituded product was obtained. Mass Spectrometry used was of the type MALDI-TOF-MS (Matrix-Assisted-Laser-Desorption-Ionozation Time-Of-Flight Mass Spectrometry).
   B) shows the initial rFab of Example 1, which comprises the rFab and a minor component/impurity consisting of a monodeamidated rFab;
   C) shows that the reaction is specific and does not take place in absence of the reducing agent.

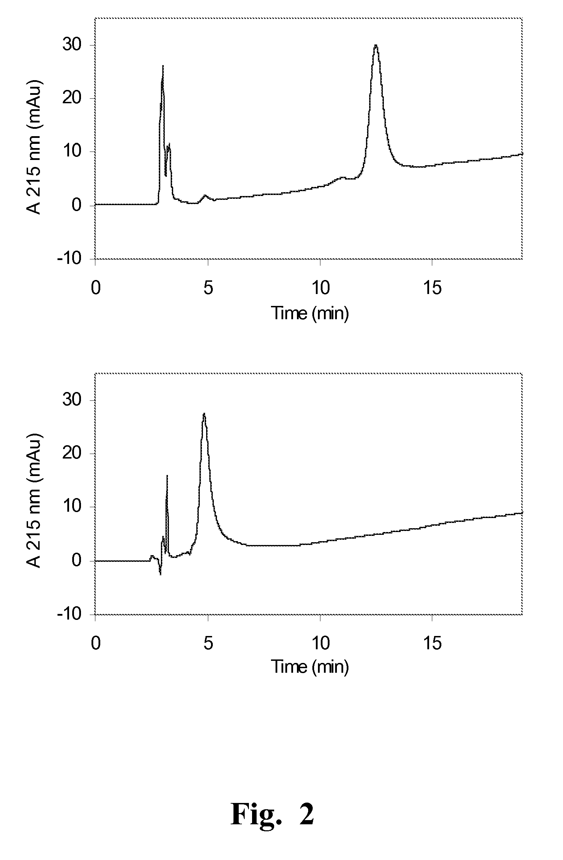

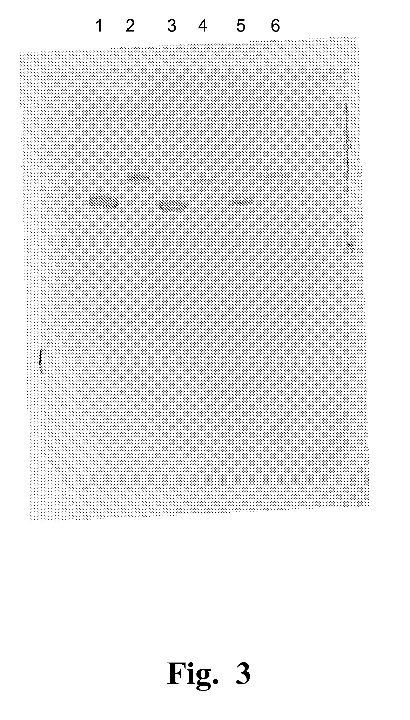

Fig. 2 shows HPLC cation-exchange analysis of the rFab essentially free from deamidated form, before (up) and after (bottom) exhaustive conjugation with Compound D of Example 2, corresponding to lanes 3 and 4 of Fig 3. The profiles show that the main peak shifts to lower retention times, due to conjugation, and that the purity of the preparation, once removed the reagents, is similar to that of the starting rFab (the peaks at the void volume around 3 min are due to reagents).
Fig. 3 shows native electrophoresis of rFab preparations, before and after exhaustive alkylation with Compound D of Example 2. The reaction mixtures were analyzed without any purification step. The reduction in migration distance that follows reaction with Compound D confirms the attachment of a definite number of negatively charged groups. MALDI-TPOF MS analysis confirmed that the disubstituted product was obtained.
   1. rFab (prep. 1)
   2. rFab (prep. 1) conjugated with Compound D
   3. rFab (prep. 2)
   4. rFab (prep. 2) conjugated with Compound D
   5. rFab (prep. 3)
   6. rFab (prep. 3) conjugated with Compound D

The invention is further illustrated in details by reference to the following non-limiting Examples:

### Example 1

Reduction and alkylation of a recombinant anti-*Herpes simplex* virus Fab with β-maleimidopropionic acid.

A model reaction system was established in order to test several reaction conditions and to easily characterize the reaction products. A commercially available maleimido derivative endowed with a ionizable group, β-maleimidopropionic acid (following compound of formula I), was
selected as a model compound, which allowed the evaluation of the number of conjugated moieties by a simple ion-exchange chromatography
analysis, together with MS analysis

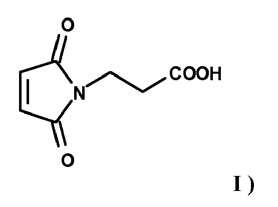

### The optimised procedure was the following one:

One volume V of a 2 mM TCEP solution was prepared by 1 to 250 dilution of the 0.5 M commercial product (Pierce) in a thoroughly deareated buffer containing 50 mM Tris-HCl, 5 mM EDTA at pH = 7.0. Then, this solution was added to an equivalent volume V of a 10 µM solution of the rFab of the title (prepared according to the previously mentioned Cattani P et al. reference) and incubated for 30 min at 37 °C. Then, a V/2 volume of 50 mM β-maleimidopropionic acid in 0.1 M acetate buffer at pH = 5 was added and the reaction mixture was kept 1 h at 37 °C. At this point the reaction is complete and, if required, excess reactants can be removed by conventional separation procedures, like dialysis or gel-filtration.

For analytical purposes, a sample was injected into a cation-exchange HPLC column and eluted with a salt gradient. Chromatography was performed on a WP Carboxy-sulfon column (J.T. Baker) at 1 mL/min, using a 15-min gradient from 60 to 120 mM phosphate buffer pH 5.8. Detection was performed at 215 nm. The results are shown in Fig. 1 and demonstrate that the rFab is completely converted in a homogeneous product having 2 more negative charges (Fig. 1A), therefore corresponding to the disubstituted derivative. MS analysis confirmed the disubstitution. The same initial rfab, unconjugated, is shown in Fig. 1B, together with a small peak eluting at 8.8 min, which is due to a mono-deamidated form of the rFab and is a useful marker of the elution position of rFab species differing only by 1 charge. Also this species is subjected to reduction and alkylation and gives the corresponding disubstituted derivative eluting at 6.38 min in Fig. 1A. Fig. 1C shows the content of a reaction mixture in which the reduction step was omitted, i.e. the reducing agent TCEP was not added. The unmodified elution profile, with respect to the unconjugated rFab of Fig. 1B, demonstrates that the alkylation is specific for thiol groups and does not take place if disulfide bonds are intact.

The rFab of Example 1, after selective reduction of the inter-chain disulfide bond, was reacted with a novel maleimido derivative of diethylentriaminopentaacetic acid (DTPA), which is a well known and widely used chelating agent of proven diagnostic and therapeutic utility (Compound D of Example 2), to give the conjugation products.

### Example 2

### Synthesis of N²,N²-bis [2-[bis(carboxymethyl)amino]ethyl]-N⁶-[4-(2,5-dioxo-1H pyrrol-1-yl)-1-oxobutyl]-L-lysine (Compound D).

The compound of the title was synthesised starting from compound A (which was prepared according to "Anelli, P.L. et al.; Bioconjugate Chem. 1999, 10, 137-140") following the two steps scheme below:

### First step:

Isobutyl chloroformate (15 mmol) was dropped into a solution of 4-maleimidobutyric acid of commercial source (Compound B; 13.6 mmol) and triethylamine (15 mmol) in tetrahydrofuran (55 mL) at -15 °C, under nitrogen atmosphere. After 15 min, a solution of compound A (13.6 mmol), prepared as previously disclosed, in tetrahydrofuran (20 mL) was dropped therein, while keeping the temperature at -4 °C. After 15 min cooling was interrupted and the mixture was stirred at room temperature for 1 h, then evaporated under vacuum. The residue was dissolved in ethyl acetate (50 mL), then washed with water. The organic phase was then dried over sodium sulphate, filtered and evaporated under vacuum.

The residue was purified by flash chromatography in an ethyl acetate/petroleum ether mixture, to obtain Compound C as a yellow oil (yield: 8.12 mmol, equivalent to 60 %). The analytical data are consistent with the desired structure.

### Second step:

Trifluoroacetic acid (68.6 mmol) was added to a solution of Compound C (6.25 g, 6.86 mmol) in dichloromethane (100 mL). After 15 h the solution was evaporated under vacuum and the residue was taken up into a further 10 ml of trifluoroacetic acid. After 6 h, the mixture was 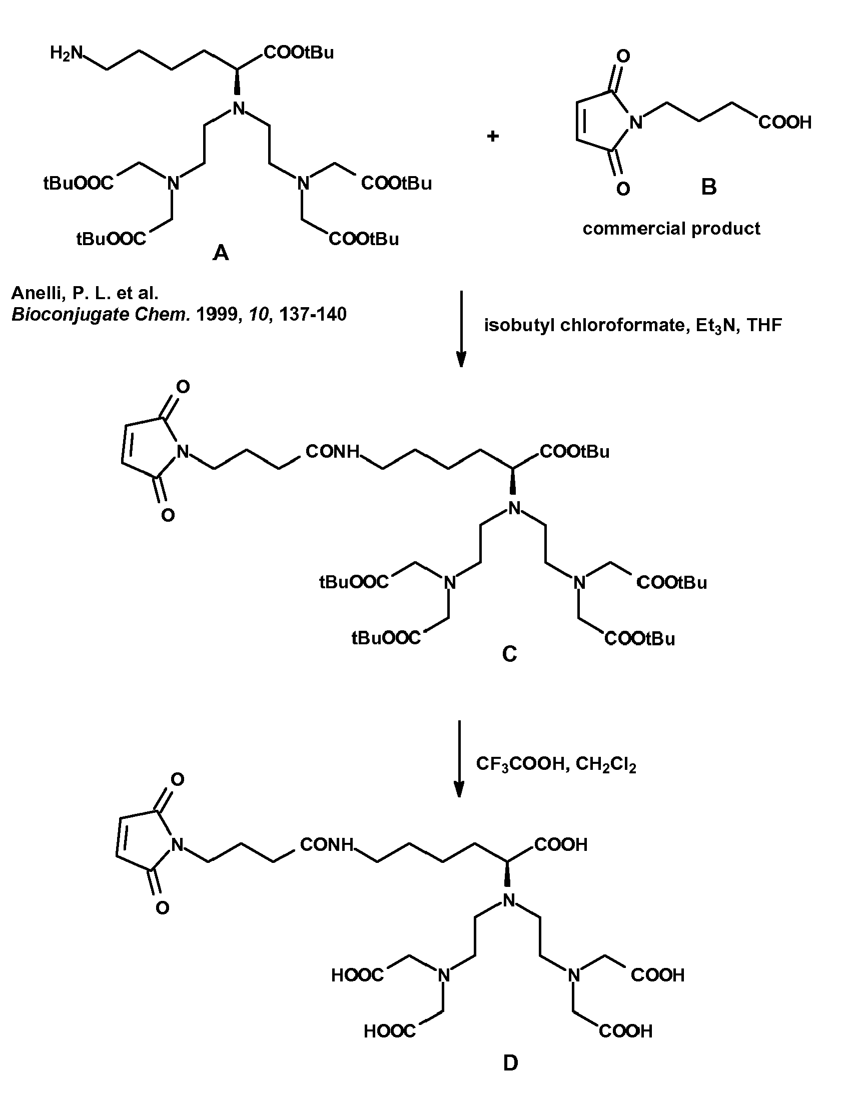 evaporated again, the residue was taken up into 50 mL of water, purified on an Amberlite XAD 16.00T column with a water/acetonitrile mixture and the relevant fractions were evaporated, to obtain Compound D as a white solid (59 % yield). The analytical data are consistent with the suggested structure

### Example 3

Selective reduction of the rFab inter-chain disulfide

Reduction of one volume V of the 10 µM rFab solution of Example 1 was carried out with an equivalent volume V of a 5 mM TCEP solution in 100 mM acetate buffer at pH = 5, for 1 h at 37 °C. As much oxygen as possible was removed from the reaction medium by bubbling nitrogen through the buffering agent before use. Under these conditions, the reduction of the inter-chain disulfide was substantially complete, as observed by SDS-PAGE analysis. However, rFab conformation was not lost as evidenced by the fact that, removing the reducing agent and incubating the reduced rFab for 2 h in 0.1 M Tris-HCl at pH = 8, the inter-chain disulfide was formed again. This fact is very important, meaning that the final product will maintain the capability of recognising the reactive site of the antigen.

### Example 4

Diconjugation of Compound D with the reduced rFab.

The cysteines formed as a consequence of the specific reduction of Example 3 were subjected to conjugation with Compound D od Example 2, directly in the same reaction medium of the reduction, by simply adding a half volume V/2 of a 100 mM Compound D solution in 0.5 M sodium acetate (final pH of the reaction solution of about 5) and incubating for 16 h at 30 °C. Under the described conditions, a single product formed in quantitative yield as shown in Fig. 2, where HPLC cation-exchange analysis of rFab before (up) and after (bottom) conjugation with Compound D is reported.

Chromatography was performed on a WP Carboxy-sulfon column (J.T. Baker) at 1 mL/min, using a 15-min gradient from 60 to 120 mM phosphate buffer pH 5.8. Detection was performed at 215 nm.

MS analysis of the final product confirmed the formation of the diconjugate.

### Example 5

Characterisation of the diconjugate of the reduced rFab with Compound D.

As the introduction of each DTPA derivative molecule causes an increase in the protein total negative charge, protein charge analysis may be used to evaluate the homogeneity of the preparation, i.e. to make sure that no conjugates with a variable number of DTPA derivatives are formed, thus leading to a mixture of different compounds. Charge analysis can be carried out by electrophoretic and chromatographic techniques. The used electrophoretic technique was native electrophoresis. According to this technique, protein migration depends on both molecular weight and charge, however, for the reduced rFab before and after conjugation with Compound C, being the protein mass substantially the same, the difference of the electrophoretic run was due solely to the differences in the charge introduced by the DTPA derivative. As shown in Fig. 3, three preparations of rFab analysed before and after conjugation with Compound D showed the same behaviour, i.e. a reduction of the migration distance towards the cathode, following conjugation. The same analysis showed that the conjugation product was homogeneous and reproducible.

### Example 6

Monoconjugation of the reduced rFab with Compound D.

The inter-chain disulfide bond of the rFab of Example 1 (one volume V of the 10 µM rFab solution) was selectively reduced as described in Example 3, then a half volume V/2 of a 0.5 mM Compound D solution in 0.5 M sodium acetate (final pH of the reaction solution of about 5) was very slowly dropped directly into the same reaction medium of the reduction at about 5 °C and the condensation was continuously monitored by HPLC.

The formation of the product of mono-alkylation was shown by the appearance of an increasing peak of intermediate retention time between the unreacted reduced rFab and the diconjugation product. When the area of this peak became bigger than the ones of the other two products, the reaction was stopped and the final mixture was purified by chromatography. MS analysis confirmed that the main peak corresponded to the monoalkylated compound.

## Claims

1. A process for the preparation of a chemical conjugate between an immunoglobulinFab fragment and molecular entities imparting diagnostic utility selected from derivatives of chelating agents for, or chelates of, radionuclides, paramagnetic metal ions or luminescent metal ions, a chromophoric fluorescent or a phosphorescent molecule, a lipophilic chain bearing molecular entity incorporated into liposomes, phospholipid-stabilized microbubbles, triglyceride- or polymer-based microspheres, microballoons which carry the diagnostic agent, whereby the only sites of conjugation on the Fab fragment are one or both of the sulfhydryl groups deriving from the selective and quantitative reduction of the inter-chain disulfide bond of said Fab fragment and whereby said one molecular entity or said two molecular entities imparting diagnostic utility have at least one free sulfhydryl-reactive group, **characterized in that** the conjugation stoichiometric molar ratio molecular entity to Fab fragment is in the range from 0.95 to 1.05 or in the range from 1.95 to 2.05, the process comprising:
a) the selective and quantitative reduction of the inter-chain disulfide bond of a Fab fragment to give two free sulfhydryl groups, under the following conditions:
| | |
|---|---|
| Fab concentration | 1-100 µM |
| TCEP | 0.1-10 mM |
| pH of the buffered solution | 4-8 |
| Reaction time | 5-180 min |
| Reaction temperature | 4-45 °C |
and
b) the quantitative functionalization of one or both of the sulfhydryl groups from step a) with one or two molecular entity or entities, respectively, having at least one free sulfhydryl-reactive group and imparting diagnostic utility, to give mono- or diconjugate compounds, said diconjugates deriving from either symmetric or asymmetric functionalization of the sulfhydryl groups;
wherein said quantitative functionalisation is performed immediately at the end of the reduction step a), in the same reaction medium, by adding a buffered aqueous solution of the conjugating molecular entity, without purifying the reduced Fab fragments.

2. The process of claim 1, wherein step a) is performed under the preferred conditions:
| | |
|---|---|
| Fab concentration | 1.5-10 µM or 1-5 µM |
| TCEP | 0.5-5 mM |
| pH of the buffered solution | 5-7 |
| Reaction time | 25-70 min |
| Reaction temperature | 25-40 °C |

3. The process of claims 1 or 2, wherein the final buffered aqueous reaction solution has the following characteristics:
| | |
|---|---|
| Fab concentration | 2-5 µM |
| TCEP | 0.5-5 mM |
| Conjugating moiety concentration | 0.1-100 mM |
| pH of the buffered solution | 5-7 |
| Reaction time | ≥30 min |
| Reaction temperature | 4-45 °C or 20-40 °C |
